# EUROPÄISCHE PATENTSCHRIFT

(11) **EP 3 579 911 B1**
(45) Veröffentlichungstag und Bekanntmachung des Hinweises auf die Patenterteilung: **13.05.2020**
(21) Anmeldenummer: 18717216.8
(22) Anmeldetag: 14.03.2018
(51) Int. Cl.: A61N 1/04

(54) **EMS-KLEIDUNGSSTÜCK**
EMS GARMENT
VÊTEMENT EMS

(30) Priorität: 05.04.2017 DE 102017003321
(43) Veröffentlichungstag der Anmeldung: 18.12.2019
(73) Patentinhaber: Miha Bodytec GmbH, 86368 Gersthofen (DE)
(72) Erfinder: DECKER, Jürgen, 86551 Aichach (DE); KÖRNER, Arno, 82211 Herrsching (DE)
(74) Vertreter: Munk, Ludwig Hubert
(86) Internationale Anmeldenummer: PCT/EP2018/025062
(87) Internationale Veröffentlichungsnummer: WO 2018/184728

(56) Entgegenhaltungen:
- WO-A1-2014/090927
- CN-B- 105 476 098
- DE-U1-202016 105 258
- FR-A1- 2 758 268
- JP-A- 2009 005 993
- US-A1- 2015 202 429
- US-A1- 2016 303 363

## Beschreibung

Die Erfindung betrifft ein EMS-Kleidungsstück gemäß dem Oberbegriff des Anspruchs 1, wie es zur Durchführung eines EMS-Trainings benötigt wird.

EMS steht dabei für Elektro-Muskel-Stimulation, auch Elektro-Myo-Stimulation genannt. Dabei werden Muskeln im lebenden Körper zu Muskelaufbauzwecken in Fitness-Studios oder bei Personal-Trainern mit elektrischen Reizen beaufschlagt, um die Muskeln zu kräftigen. Dabei werden großflächige, im Regelfall textile Elektroden, im folgenden EMS-Elektroden genannt, verwendet. Denn anders als bei medizinischen Anwendungen wie beispielsweise Defibrillatoren müssen nicht extrem hohe Spannungen übertragen werden, sondern es geht um eine gute Anlage der Elektroden am Körper, Hygiene bei wechselnden Benutzern und Komfort beim Anlegen von mit den EMS-Elektroden versehenen EMS-Kleidungsstücken. Die für die Elektro-Muskel-Stimulation von entsprechenden Reizerzeugungseinheiten an die Elektroden gesandten Spannungsstöße haben dabei eine um 10er-Potenzen geringere Amplitude als solche, die bei Defibrillatoren oder ähnlichem eingesetzt werden. So liegen die Scheitelwerte der Ausgangsspannung im EMS-Bereich in der Regel bei 70 bis 160 Volt bei einer Stromstärke von 10 bis 20 mA.

Es werden also in der EMS-Reizerzeugungseinheit getaktete Impulsströme erzeugt und über Stromleitungen zu EMS-Elektroden und über die EMS-Elektroden durch den Körper hindurch geleitet. Dabei werden im Regelfall die EMS-Elektroden paarweise so geschaltet, dass bei einem Zeitpunkt eine erste EMS-Elektrode mit dem Strom impulsartig beaufschlagt wird und eine zweite EMS-Elektrode zu diesem Zeitpunkt nicht und den durch den Körper geleiteten Strom abfließen lässt. Beim Impulswechsel wird die zweite EMS-Elektrode mit Strom impulsartig beaufschlagt und die erste EMS-Elektrode nicht. Der Stromlauf ist bei einer Bekleidung mit mehreren EMS-Elektrodenpaaren z.B. Brust, Bauch, Rücken Oberarme, Oberschenkel etc. noch komplexer.

Die EMS-Elektroden sind mit der EMS-Reizerzeugungseinheit, also einem Steuergerät verschaltet, das beispielsweise in einem Frequenzbereich von 2 bis 150 Hz mit einer Impulsbreite von 50 bis 400 Mikrosekunden und einer Impulspause von 0 bis 10 Sekunden arbeitet. Der max. Scheitelwert der elektrischen Ausgangsspannung liegt beispielsweise bei 70 bis 160 V bei einer Stromstärke von ca. 10 bis 20 mA.

Die deutsche Gebrauchsmusterschrift DE 20 2013 104 554 U1 offenbart eine EMS-Weste, bei der die Elektroden per Klettverbindung auf der Körpervorderseite und der Körperrückseite zugewandte Flächenabschnitte aus Neopren aufgebracht sind. Die Flächenabschnitte sind dabei durch den Körperflanken zugewandte Streifen aus einem sehr dehnbaren Material und mittels einer Vielzahl von Zugriemen bzw. Zurrgurten verbunden, wobei die Fixierung der Weste am Körper des Trainierenden mittels der Zurrgurte zu erfolgen hat, was aufwändig ist.

Die US-Patentschrift US 7,072,721 B1 offenbart ebenfalls eine EMS-Weste mit per Klettverschluss anbringbaren Elektroden, jedoch ohne lästige Zurrgurte, schweigt jedoch zum Aufbau und zum Material der Weste.

Eine EMS-Elektrode ist beispielsweise der deutschen Patentanmeldung DE 10 2007 046 886 A1 zu entnehmen. Mit derartigen EMS-Elektroden versehene EMS-Kleidungsstücke werden vor dem Training genässt und in der Regel über einem Unterbekleidungstück (z. B. Stretch-T-Shirt) getragen. Ein für ein EMS-Training verwendeter Satz von EMS-Elektroden besteht beispielsweise aus an einer Weste angebrachten Elektroden zur Stimulierung der Rumpfmuskulatur, aus zwei an einem Po-Gurt angebrachten Elektroden, sowie zwei Oberarmgurten und zwei Oberschenkelgurten mit EMS-Elektroden. Um den Tragekomfort des EMS-Kleidungsstücks zu erhöhen und seine Anlage an den Körper zu optimieren bestehen solche Kleidungsstücke oft aus dehnbaren Textilien mit Elastanfasern, wobei auch die EMS-Elektroden häufig aus textilen Materialien mit eingewebten Elastanfasern gebildet werden, um einen guten Sitz am Körper zu gewährleisten. Ein Beispiel für ein solches EMS-Kleidungsstück ist dem deutschen Patent DE 10 2009 017 179 B4 zu entnehmen.

Nachteilig daran ist es, dass derartige, aus dehnbaren Textilien bestehenden EMS-Kleidungsstücke mit der Zeit ausleiern.

Es gibt auch textilintegrierte EMS-Elektroden, die insgesamt oder zumindest deren Stromübertragungsbereich in das Trägertextil eingearbeitet sind, wobei neben den Elektroden auch die an die Elektroden angeschlossenen Leiterbahnen in das EMS-Kleidungsstück integriert sind. So zeigen die deutsche Patentanmeldung DE 10 2012 112 153 A1 und die internationale Patentanmeldung WO 2014/000736A2 textilintegrierte EMS-Elektroden, deren Stromübertragungsbereich als Inselintarsie in ein Flachgestrick eingearbeitet und durch eine ebenfalls eingestrickte Anschlussleitung, die in einem ebenfalls eingestrickten Kanal verläuft, mit einem Stromanschluß verbunden ist.

Insbesondere bei derartigen, textilintegrierten EMS-Elektroden liegt ein besonderer Augenmerk darauf, dass sie auch eine gewisse Elastizität aufweisen müssen, da die EMS-Kleidungsstücke und mit ihnen die EMS-Elektroden eng am Körper anliegen sollen. Die Elastizität der EMS-Elektroden lässt sich mit den für die Gestricke verwendeten, leitfähigen Fäden nicht oder nur mit teuren Spezialfäden, die gleichzeitig leitfähig und elastisch sind, erreichen.

Weiterhin existieren im Bereich NMES (neuromuskuläre Stimulation) Kleidungsstücke aus Neopren, die mit Taschen bestückt sind, in denen Elektroden angeordnet sind, siehe US 2016/0303363 A1.

Hiervon ausgehend ist es Aufgabe der vorliegenden Erfindung ein EMS-Kleidungsstück mit verbesserter Haltbarkeit und Passform zu schaffen, welches in der Fertigung kostengünstig ist.

Diese Aufgabe wird mit den Merkmalen des Anspruchs 1 gelöst.

Erfindungsgemäß wird ein EMS-Kleidungsstück zum Umschließen eines Körperteils eines Trainierenden während einer EMS-Trainingseinheit vorgeschlagen, das beispielsweise als Weste oder Po-Gurt ausgebildet sein kann. Das EMS-Kleidungsstück ist dabei mit einer Mehrzahl EMS-Elektroden zur Übertragung von aus Stromimpulsen und/oder Wechselstrom mit vorgegebenen Werten wie Amplitude und Frequenz bestehenden EMS-Reizen von einer angeschlossenen EMS-Reizerzeugungseinheit auf den umschlossenen Körperteil ausgestattet. Weiterhin weist das EMS-Kleidungsstück Textilflächenabschnitte auf, die während des Trainings flächig an dem Körperteil anliegen. Die EMS-Elektroden sind zur Sicherstellung einer guten Anlage an dem Körperteil als ein Pad oder Textilstrukturabschnitt mit einer elektrisch leitenden Leitschicht, ausgebildet, so dass sie sich flexibel an den Körperteil anlegen, und auf einem oder mehreren der Textilflächenabschnitte angebracht.

Das erfindungsgemäße EMS-Kleidungsstück zeichnet sich dabei dadurch aus, dass in einer Vorderseite und einer Rückseite des Körperteils während des Trainings zugewandten Bereichen jeweils zumindest einer der Textilflächenabschnitte am Kleidungsstück angeordnet ist, wobei die der Vorderseite und der Rückseite zugewandten Textilflächenabschnitte über Dehnungsabschnitte verbunden sind, die aus einem gummielastischen, nicht textilen Material bestehen und vorzugsweise den beiden Flanken des Körperteils zugewandt sind.

Der bzw. die Dehnungsabschnitte können dabei aus einem elastomeren Vollmaterial bestehen, wobei sich insbesondere vulkanisierter Silikonkautschuk im Hinblick auf die benötigte Kompression auf den zu umschließenden Körperteil und auf die dafür nötige Dehnfähigkeit und elastische Rückstellkraft als besonders geeignet herausgestellt hat.

Es wäre aber auch denkbar, dDie Dehnungsabschnitte nach Art eines Korsetts jeweils durch ein gummielastisches Band auszubilden, welches durch Ösen am Rand der beiden miteinander zu verbindenden Textilflächenabschnitte geführt ist, um die für die langfristige Passform des EMS-Kleidungsstücks notwendige Dauerelastizität bereit zu stellen.

Gegenüber EMS-Kleidungsstücken, bei denen die Passform bzw. Anlage den zu trainierenden Körperteil mittels dehnbaren Textilien, wie beispielsweise Elastan oder mit Elastanfasern versehenen Geweben hergestellt wird, lassen sich mit aus gummielastischen, nicht textilen Materialien bestehenden Dehnungsabschnitten deutlich haltbarere EMS-Kleidungsstücke herstellen, die bei Beanspruchung nicht so schnell ausleiern und beim Waschen auch bei aus hygienischen Gründen erwünschten, hohen Temperaturen ebenfalls ihre Elastizität nicht verlieren. Zudem sind derartige Dehnungsabschnitte kostengünstig herstellbar und ermöglichen einen insgesamt kostengünstigen Aufbau des EMS-Kleidungsstücks, weil Einstellgurte entfallen können.

Zudem entfällt die Notwendigkeit des Einsatzes von elastischen EMS-Elektroden. Auch wenn beim erfindungsgemäßen EMS-Kleidungsstück aufgrund angenehmerer Trageeigenschaften bevorzugt textile EMS-Elektroden zum Einsatz kommen wäre damit grundsätzlich auch die Rückkehr zu den in früheren Zeiten als EMS-Elektroden verwendeten, günstigen Polymer-Pads, die mit leitfähigen Partikeln (z.B. Ruß) gefüllt sind, denkbar, wie sie beispielsweise der deutschen Patentschrift DE 20 18 239 C2 zu entnehmen sind.

Im Rahmen der Erfindung wäre es dabei auch denkbar, nur an einer Körperflanke einen Dehnungsabschnitt aus dem gummielastischen, nicht textilen Material vorzusehen, oder sogar den Dehnungsabschnitt in den Rückenbereich zu verlegen.

Besonders bevorzugt ist es jedoch, wenn jeder der Dehnungsabschnitte jeweils als ein sich flächig erstreckendes Flächenelement ausgebildet ist. Es können also beispielsweise eine Mehrzahl von flächigen Dehnstreifen aus gummielastischem Material entlang jeder Körperflanke von unten nach oben vorgesehen sein, um den Textilflächenabschnitt an der Rückenpartie mit den beiden jeweils als textiler Flächenabschnitt ausgebildeten Vorderhälften einer EMS-Weste zu verbinden. Wesentlich weniger anfällig und leichter an den Textilflächenabschnitten zu befestigen ist jedoch ein einstückiges Flächenelement. Daher ist bevorzugt an jeder der beiden Körperflanken ein als einstückiges Flächenelement ausgeformter Dehnungsabschnitt vorgesehen um dort den rückseitigen Textilflächenabschnitt an seiner den Dehnungsabschnitt zugewandten Kante mit einem vorderseitigen Textilflächenabschnitt an seiner zugewandten Kante zu verbinden.

Bei der Ausgestaltung der Dehnungsabschnitte muss dabei darauf geachtet werden, dass diese für die Dauerbelastung mit Zuglasten während des EMS-Trainings und Temperatur- und Reibungsbelastungen während der Waschgänge ausgelegt sind und daher eine gewisse Grundrobustheit an den Tag legen. Daher weisen die Dehnungsabschnitte bevorzugt Löcher auf, über welche eine erhöhte Dehnfähigkeit bei reduzierter elastischer Rückstellkraft in Zugrichtung beim Umschließen des EMS-Kleidungsstücks um den Körperteil herum bereitgestellt wird, gegenüber einem Dehnungsabschnitt ohne Löcher. Zwar könnte der Dehnungsabschnitt auch einfach dünner gemacht werden, wodurch dieser jedoch anfälliger würde. Weiterhin bevorzugt ist es dabei, wenn die Löcher eine kreisrunde oder noch besser ovale Form aufweisen, mit Längsachse des Ovals in Zugrichtung, so dass sich eine besonders gleichmäßige Kraftverteilung im Dehnungsabschnitt erreichen lässt.

Besonders vorteilhaft wirken sich die erfindungsgemäß vorgesehenen gummielastischen Dehnungsabschnitte dabei dann aus, wenn an dem EMS-Kleidungsstück, welches die nötige Elastizität ja nun mittels der gummielastischen Dehnungsabschnitte aufweist, ansonsten auf elastanhaltige Textilien oder die Verwendung ähnlicher, elastisch dehnbarer Garne oder Web- bzw. Stricktechniken für die Textilflächenabschnitte verzichtet wird und diese anstatt dessen aus einem nicht elastisch dehnbaren, elastanfreien Textilmaterial gefertigt werden. Dadurch kann das bereits beschriebene Ausleiern des EMS-Kleidungsstücks vermieden werden und damit nicht nur ein hoher Verschleiß, sondern auch die üblicherweise nötigen Nachstellgurtbänder, mit denen herkömmlich ein EMS-Kleidungsstück am zu trainierenden Körperteil festgezurrt werden muss, weil bei rein textilen EMS-Kleidungsstücken ansonsten die für ein effektives Training nötige Kompression bzw. Anpresskraft sonst nicht bei jedem Körperbau des Trainierenden und in jeder Abnutzungsphase als EMS-Kleidungsstücks herstellbar ist.

Weiterhin bevorzugt weisen auch die EMS-Elektroden einen nicht elastisch dehnbaren Aufbau auf, weil damit eine aufwendige Verwebung von elektrisch leitfähigen Garnen mit dehnbaren Garnen oder die Verwendung von teuren Garnen, die beide Eigenschaften haben, vermieden werden kann. Es wäre jedoch denkbar, z. B. an einem Po-Gurt, der lediglich an seinem rückseitigen Textilflächenabschnitt EMS-Elektroden trägt, vorderseitige Textilflächenabschnitte elastisch dehnbar auszugestalten.

Besteht jeder der Dehnungsabschnitte aus einem elastomeren Vollmaterial wie vulkanisierten Silikonkautschuk, so lassen sich die Dehnungsabschnitte mit den Textilflächenabschnitten auf einfache und stabile Weise dadurch verbinden, dass sie vernäht werden. Es wäre jedoch eine Verklebung oder ein Einvulkanisieren der Textilflächenabschnitte in die als elastomere Flächenelemente ausgebildeten Dehnungsabschnitte denkbar.

In vorteilhafter Ausgestaltung ist der der Rückseite zugewandte Textilflächenabschnitt dabei einstückig ausgebildet und an seinen beiden Seiten jeweils mit zumindest einem der Dehnungsabschnitte verbunden, vorzugsweise mit genau einem einstückig als elastomeres Flächenelement ausgebildeten Dehnungsabschnitt, der sich längs der Höhenrichtung des aufgerichteten Körperteils erstreckt, welches von dem EMS-Kleidungsstück umschlossen ist. Der der Vorderseite des Körperteils zugewandte Bereich des EMS-Kleidungsstücks kann dabei von zwei Textilflächenabschnitten gebildet sein, die bevorzugt in etwa gleich groß sind, also jeweils eine Hälfte der Vorderseite des EMS-Kleidungsstücks bilden und an ihrer zur Körperflanke hin weisenden Seite jeweils mit dem dortigen, zumindest einen Dehnungsabschnitt verbunden sind. Zum Umschließen des Körperteils mit dem EMS-Kleidungsstück bzw. zum Anlegen des EMS-Kleidungsstücks kann dann ein lösbarer Verschluss wie beispielsweise ein Reißverschluss oder eine oder mehrere Steckverbindungen vorgesehen sein, über die die beiden vorderseitigen Textilflächenabschnitte miteinander verbindbar sind. Bei einer EMS-Weste eignet sich hierfür besonders gut ein Reißverschluss, wobei auch eine Hakenleiste alternativ oder ergänzend hierzu denkbar wäre. Bei einem als Po-Gurt ausgestalteten EMS-Kleidungsstück könnte dagegen eine Gürtelschnalle oder eine mit Gurtbändern an den beiden vorderseitigen Textilflächenabschnitten angebrachte Steckverbindung vorgesehen sein, wie sie von Outdoor-Rucksäcken her bekannt ist.

Insgesamt ergeben sich dabei Vorteile hinsichtlich einer optimalen Passform und einer enormen Haltbarkeit des EMS-Kleidungsstücks dank der Kombination von vulkanisiertem Silikonkautschuk und Textilflächenabschnitten, sowie ein ideales Kraft-Dehnungsverhalten bei hoher Elastizität aufgrund der aus dem vulkanisierten Silikonkautschuk bestehenden Dehnungsabschnitten. Hohe elastische Rückstellkräfte sorgen dabei für einen idealen Anpressdruck der Elektroden bzw. die dafür notwendige Kompression des EMS-Kleidungsstücks auf den zu umschließenden Körperteil, ohne dass es dabei zu Materialermüdung oder Ausleiern des EMS-Kleidungsstücks im Dauereinsatz kommt. Silikonkautschuk wird dabei seit langem in medizinischen Anwendungsfeldern eingesetzt und eignet sich auch aufgrund seiner antibakteriellen Eigenschaften für den Einsatz am EMS-Kleidungsstück. Dazu kommt eine enorme Anti-Rutsch-Wirkung, welche ein Verrutschen des EMS-Kleidungsstücks während des Trainings verhindert.

Nachfolgend werden anhand der beiliegenden Zeichnungen vorteilhafte Ausführungen der Erfindung näher beschrieben. Es zeigen:
- Figur 1: einen EMS-Po-Gurt gemäß einer vorteilhaften Ausführungsform der Erfindung;
- Figur 2: eine EMS-Weste gemäß einer weiteren vorteilhaften Ausführungsform der Erfindung; und
- Figur 3: die in Figur 2 gezeigte EMS-Weste in an einer Schaufensterpuppe angelegtem Zustand.

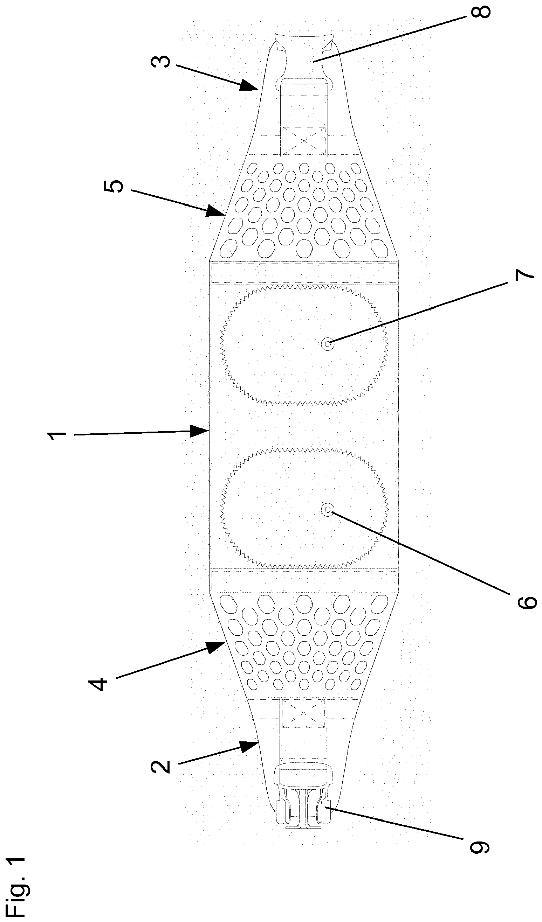

Zunächst wird Bezug genommen auf die Figur 1, welche ein als Po-Gurt ausgestaltetes EMS-Kleidungsstück zeigt. Der EMS-Po-Gurt weist an seinem im angelegten Zustand der Körperrückseite zugewandten Bereich einen Textilflächenabschnitt 1 auf, der zwei den beiden Pobacken zugeordnete EMS-Elektroden aufweist, deren zum Anschluss von Stromverbindungskabeln vorgesehenen Druckknöpfe mit 6, 7 bezeichnet sind. An seinen beiden Seitenkanten ist der rückwärtige Textilflächenabschnitt 1 jeweils mit einem flächigen, aus vulkanisierten Silikonkautschuk bestehenden, einstückig ausgeführten Dehnungsabschnitt 4, 5 vernäht. Jeder der beiden Dehnungsabschnitte 4, 5 kann dabei kreisrunde Löcher aufweisen, um die Dicke des Dehnungsabschnitts 4, 5 trotz der gewünschten Dehnfähigkeit so hoch wählen zu können, dass die Dehnungsabschnitte 4, 5 eine hohe Robustheit haben. An ihrer dem rückwärtigen Textilabschnitt 1 abgewandten Bereich an der Vorderkante sind die beiden Dehnungsabschnitte 4, 5 dabei jeweils mit einem weiteren Textilflächenabschnitt 2, 3 verbunden, vorzugsweise vernäht. Die beiden Textilflächenabschnitte 2, 3 tragen dabei jeweils Gurtbänder mit Steckelementen 8, 9, mit denen sie auf der Körpervorderseite nach dem Anlegen des Po-Gurts miteinander verbindbar sind. Die Textilabschnitte 1, 2, 3 brauchen keine Dehnfähigkeit aufzuweisen, so dass die zur passgenauen Anlage des Po-Gurts an den Körper nötige Elastizität allein über die beiden Dehnungsabschnitte 4, 5 bereitgestellt wird.

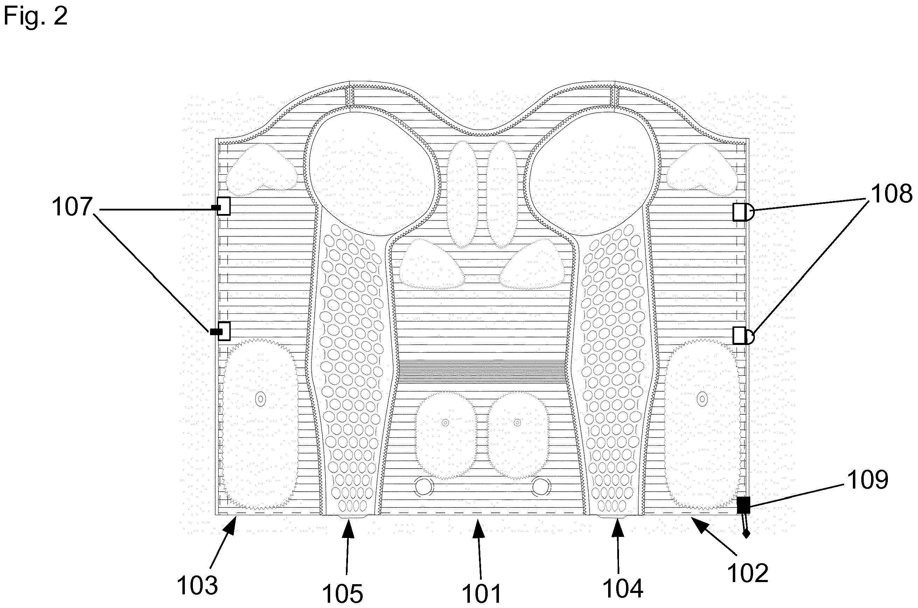

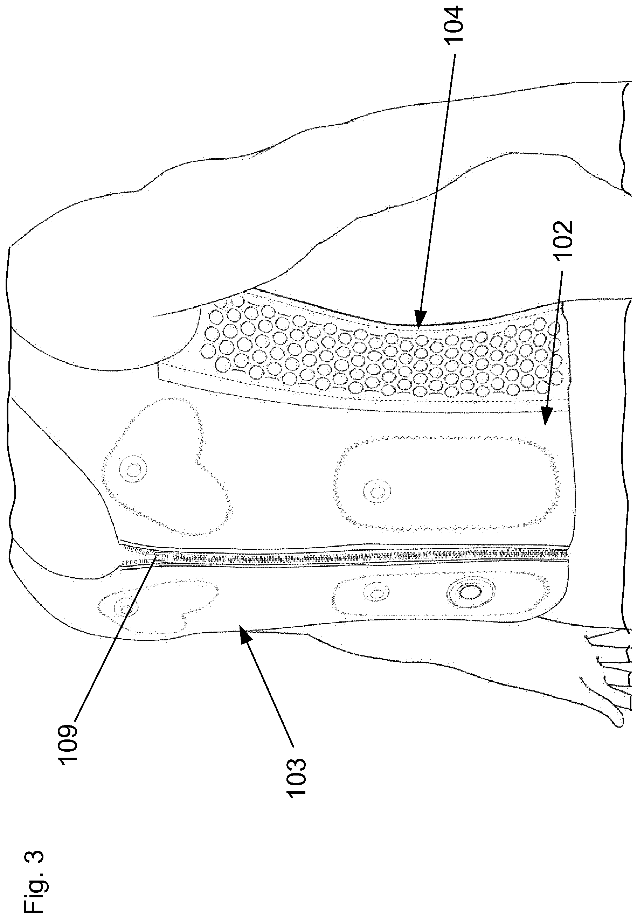

Die Figuren 2 und 3 zeigen ein als Weste mit drei Elektrodenpaaren auf der Körperrückseite und zwei Elektrodenpaaren auf der Körpervorderseite ausgebildetes EMS-Kleidungsstück. Ein der Rückenpartie des Trainierenden zugewandter, einstückiger Textilflächenabschnitt mit den drei Elektrodenpaaren ist dabei mit 101 bezeichnet und oberhalb der Armschlaufen mit zwei weiteren, zur Anlage an Körpervorderseite vorgesehenen Textilflächenabschnitten 102, 103 mit je zwei EMS-Elektroden vernäht. Unterhalb der beiden Armschlaufen erstrecken sich dabei längs der Höhenrichtung des aufgerichteten Körpers zwei einstückige, aus vulkanisiertem Silikonkautschuk bestehende Dehnungsabschnitte 104, 105, die entlang ihrer Seitenkanten mit den zugewandten Seitenkanten der jeweiligen Textilflächenabschnitte 101, 102 bzw. 101, 103 verbunden, vorzugsweise vernäht sind. Die für eine flächige Anlage der EMS-Elektroden am Rumpf des Trainierenden nötige elastische Rückstellkraft bzw. Kompression der Weste wird dabei allein von den beiden elastomeren Dehnungsabschnitten 104, 105 bereitgestellen, wenn bauchseitig ein in Richtung des aufgerichteten Körpers verlaufender Reißverschluss 109 geschlossen ist. Weiterhin weist die EMS-Weste Haken 107 einerseits und Ösen 108 andererseits auf, an denen die beiden vorderseitigen Textilflächenabschnitte 102, 103 aneinander vorfixiert werden können, bevor man den Reißverschluss 109 schließt.

Abwandlungen und Modifikationen sind möglich, ohne den Rahmen der Erfindung zu verlassen.

So könnten auch Oberarm- oder Oberschenkelbänder mit entsprechenden, vorzugsweise einstückigen, elastomeren Dehnungsabschnitten versehen sein, was die bisher zum Festzurren nötigen Einstellschlaufen und Klettverschlüsse obsolet machen würde.

## Patentansprüche

1. EMS-Kleidungsstück, wie beispielsweise Weste oder Pogurt, zum Umschließen eines Körperteils eines Trainierenden während einer EMS-Trainingseinheit, mit Textilflächenabschnitten (1, 2, 3; 101, 102, 103) zur flächigen Anlage an dem Körperteil, und mit EMS-Elektroden zur Übertragung von aus Stromimpulsen und/oder Wechselstrom mit vorgegebenen Werten wie Amplitude und Frequenz bestehenden EMS-Reizen von einer angeschlossenen EMS-Reizerzeugungseinheit auf den Körperteil, welche jeweils an einem der Textilflächenabschnitte (1, 2, 3; 101, 102, 103) angebracht sind und welche als ein Pad oder ein Textilstrukturabschnitt mit einer elektrisch leitenden Leitschicht so ausgebildet sind, dass sie sich flexibel an den Körperteil anlegen, **dadurch gekennzeichnet, dass** das EMS-Kleidungsstück in einer Vorderseite und einer Rückseite des Körperteils zugewandten Bereichen des EMS-Kleidungsstücks jeweils zumindest einen der Textilflächenabschnitte (1, 2, 3; 101, 102, 103) aufweist, wobei die der Vorderseite und der Rückseite zugewandten Textilflächenabschnitte (1, 2, 3; 101, 102, 103) über beiden Flanken des Körperteils zugewandte Dehnungsabschnitte (4, 5; 104; 105) entlang einer Höhenrichtung des aufgerichteten Körperteils verbunden sind, die aus einem gummielastischen, elastomeren Vollmaterial bestehen oder nach Art eines Korsetts jeweils durch ein gummielastisches Band ausgebildet sind, welche durch Ösen am Rand der beiden durch den Dehnungsabschnitt (4, 5; 104; 105) miteinander zu verbindenden Textilflächenabschnitte geführt sind.

2. EMS-Kleidungsstück nach Anspruch 1, **dadurch gekennzeichnet, dass** die Dehnungsabschnitte (4, 5; 104; 105) jeweils als ein sich flächig erstreckende Flächenelement ausgebildet sind, welches insbesondere mit Löchern versehen ist, beispielsweise mit kreisrunden oder ovalen Löchern.

3. EMS-Kleidungsstück nach Anspruch 1 oder 2, **dadurch gekennzeichnet, dass** an jeder der beiden Körperflanken jeweils einer der Dehnungsabschnitte (4, 5; 104; 105) vorgesehen ist, welcher einstückig ausgeformt ist.

4. EMS-Kleidungsstück nach Anspruch 1, 2 oder 3, **dadurch gekennzeichnet, dass das elastomere Vollmaterial, aus dem die Dehnungsabschnitte (4, 5; 104; 105) bestehen, ein vulkanisierter Silikonkautschuk ist.**

5. EMS-Kleidungsstück nach einem der vorhergehenden Ansprüche, **dadurch gekennzeichnet, dass** die Dehnungsabschnitte (4, 5; 104; 105) mit den Textilflächenabschnitten (1, 2, 3; 101, 102, 103) vernäht sind.

6. EMS-Kleidungsstück nach einem der vorhergehenden Ansprüche, **dadurch gekennzeichnet, dass** der der Rückseite zugewandte Textilflächenabschnitt (1; 101) einstückig ausgebildet ist und an seinen beiden Seiten jeweils mit zumindest einem der Dehnungsabschnitte (4, 5; 104; 105) verbunden ist, wobei der der Vorderseite zugewandte Bereich des EMS-Kleidungsstücks von zwei vorzugsweise in etwa gleich großen Textilflächenabschnitten (2, 3; 102, 103) gebildet wird, welche an ihrer zur Körperflanke hin weisenden Seite jeweils mit dem dortigen, zumindest einen Dehnungsabschnitt (4, 5; 104; 105) verbunden sind und welche zum Umschließen des Körperteils mit dem EMS-Kleidungsstück über einen lösbaren Verschluss wie beispielsweise einen Reißverschluss (109) oder eine Steckverbindung (8, 9) miteinander verbindbar sind.

7. EMS-Kleidungsstück nach einem der vorhergehenden Ansprüche, **dadurch gekennzeichnet, dass** zumindest der der Rückseite zugewandte Textilflächenabschnitt (1; 101), bevorzugt auch die vorderseitigen Textilflächenabschnitte (2, 3; 102, 103) EMS-Elektroden tragen.

8. EMS-Kleidungsstück nach einem der vorhergehenden Ansprüche, **dadurch gekennzeichnet, dass** die EMS-Elektroden einen nicht elastisch dehnbaren Aufbau aufweisen.

9. EMS-Kleidungsstück nach einem der vorhergehenden Ansprüche, **dadurch gekennzeichnet, dass** zumindest jeder EMS-Elektroden tragende Textilflächenabschnitt (1, 2, 3; 101, 102, 103) aus einem nicht elastisch dehnbaren, elastanfreien Textilmaterial besteht.

## Claims

1. An EMS garment, such as a vest or a bottom strap, for enclosing a body part of a trainee during an EMS training session, comprising textile area sections (1, 2, 3; 101, 102, 103) for plane snuggeling to the body part, and EMS electrodes for transmitting EMS stimuli consisting of current pulses and/or alternating current with predetermined values such as amplitude and frequency from a connected EMS stimulation generating unit to the body part, which EMS electrodes are each connected to one of the textile area sections (1, 2, 3; 101, 102, 103) and are each formed as a pad or a textile structure section having an electrically conductive conductive layer so as to flexibly snuggle to the body part, **characterized in that** the EMS garment comprises, in a region facing the front side of the body part and in a region facing the rear side of the body part, at least one of the textile area sections (1, 2, 3; 101, 102, 103), respectively, wherein the textile area sections (1, 2, 3; 101, 102, 103) facing the front side and the rear side are connected along a height direction of the erected body part by stretch sections (4, 5; 104; 105) facing both flanks of the body part, each of which consists of a rubber-elastic, elastomeric solid material or is formed in the manner of a corset by a rubber-elastic band which is guided through eyelets at the edges of the two textile area sections (1, 2, 3; 101, 102, 103) to be connected to one another by the stretch sections (4, 5; 104; 105).

2. EMS garment according to claim 1, **characterized in that** the stretch sections (4, 5; 104; 105) are each designed as a planarly extending flat element which is provided in particular with holes, for example with circular or oval holes.

3. EMS garment according to claim 1 or 2, **characterized in that** on each of the two body flanks one of the stretch sections (4, 5; 104; 105) is provided and being formed in one piece.

4. An EMS garment according to claim 1, 2 or 3, **characterized in that** the elastomeric solid material of which the stretch sections (4, 5; 104; 105) consist is a vulcanized silicone rubber.

5. An EMS garment according to one of the preceding claims, **characterized in that** the stretch portions (4, 5; 104; 105) are sewn to the textile area sections (1, 2, 3; 101, 102, 103).

6. EMS garment according to one of the preceding claims, **characterized in that** the textile area section (1; 101) facing the rear side is formed in one piece and is connected on each of its two sides to at least one of the stretch sections (4, 5; 104; 105), the region of the EMS garment facing the front side being formed by two textile area sections (2, 3; 102, 103), preferably of approximately equal size, which on their side facing the respective body flank are each connected to the at least one stretch portion (4, 5; 104; 105) there and which are connectable to one another for enclosing the body part with the EMS garment via a detachable closure such as a zipper (109) or a plug-in connection (8, 9).

7. EMS garment according to one of the preceding claims, **characterized in that** at least the textile area section (1; 101) facing the rear side, preferably also the textile area sections (2, 3; 102, 103) on the front side, carry EMS electrodes.

8. EMS garment according to one of the preceding claims, **characterized in that** the EMS electrodes have a structure not being elastically stretchable.

9. EMS garment according to one of the preceding claims, **characterized in that** at least each textile area section (1, 2, 3; 101, 102, 103) carrying EMS electrodes consists of an elastane-free textile material not being non elastically stretchable.

## Revendications

1. Vêtement EMS, tel que gilet ou ceinture fessière, destiné à être posé autour d'une partie du corps d'une personne qui s'entraîne pendant une unité d'entraînement EMS, comprenant des sections de surface textile (1, 2, 3; 101, 102, 103) destinées à être appliquées de manière plane à la partie du corps, ainsi que des électrodes EMS qui sont destinées à transmettre, d'une unité connectée de génération de stimuli EMS à la partie du corps, des stimuli EMS constitués d'impulsions de courant et/ou de courant alternatif ayant des valeurs prédéterminées telles que l'amplitude et la fréquence, et sont montées chacune sur l'une des sections de surface textile (1, 2, 3; 101, 102, 103) et qui sont conçues en tant que pad ou section de structure textile avec une couche conductrice électriquement conductrice de telle sorte qu'elles s'appliquent de manière flexible contre la partie du corps, **caractérisé par le fait que** le vêtement EMS présente respectivement au moins l'une des sections de surface textile (1, 2, 3; 101, 102, 103) dans des zones du vêtement EMS qui montrent vers une face avant et vers une face arrière de la partie du corps, dans lequel les sections de surface textile (1, 2, 3; 101, 102, 103) montrant vers les faces avant et arrière sont reliées entre elles le long d'une direction de hauteur de la partie du corps redressée, par des sections d'extension (4, 5; 104; 105) qui montrent vers les deux flancs de la partie du corps et qui sont réalisées à partir d'un matériau plein élastomère et élastique comme le caoutchouc ou sont conçues chacune à la manière d'un corset par une bande élastique comme le caoutchouc que l'on fait passer à travers des œillets situés sur le bord des deux sections de surface textile à relier l'une à l'autre par la section d'extension (4, 5; 104; 105).

2. Vêtement EMS selon la revendication 1, **caractérisé par le fait que** les sections d'extension (4, 5; 104; 105) sont conçues chacune comme un élément de surface s'étendant en nappe qui, en particulier, est pourvu de trous, par exemple de trous circulaires ou ovales.

3. Vêtement EMS selon la revendication 1 ou 2, **caractérisé par le fait que** sur chacun des deux flancs du corps est prévue respectivement l'une des sections d'extension (4, 5; 104; 105) qui est formée en une seule pièce.

4. Vêtement EMS selon la revendication 1, 2 ou 3, **caractérisé par le fait que** le matériau plein élastomère à partir duquel sont réalisées les sections d'extension (4, 5; 104; 105) est un caoutchouc de silicone vulcanisé.

5. Vêtement EMS selon l'une quelconque des revendications précédentes, **caractérisé par le fait que** les sections d'extension (4, 5; 104; 105) sont cousues aux sections de surface textile (1, 2, 3; 101, 102, 103).

6. Vêtement EMS selon l'une quelconque des revendications précédentes, **caractérisé par le fait que** la section de surface textile (1; 101) tournée vers la face arrière est formée en une seule pièce et est reliée sur ses deux côtés respectivement à au moins l'une des sections d'extension (4, 5; 104; 105), dans lequel la zone du vêtement EMS qui est tournée vers la face avant est formée de deux sections de surface textile (2, 3; 102, 103) qui sont de préférence à peu près de la même taille et qui, sur leur côté montrant vers le flanc du corps, sont reliées respectivement à ladite au moins une section d'extension (4, 5; 104; 105) y présente et qui, pour poser le vêtement EMS autour de la partie du corps, peuvent être reliées les unes aux autres par une fermeture amovible, telle que, par exemple, une fermeture à glissière (109) ou une liaison mâle-femelle (8, 9).

7. Vêtement EMS selon l'une quelconque des revendications précédentes, **caractérisé par le fait qu'**au moins la section de surface textile (1; 101) montrant vers la face arrière, de préférence également les sections de surface textile (2, 3; 102, 103) face avant, portent des électrodes EMS.

8. Vêtement EMS selon l'une quelconque des revendications précédentes, **caractérisé par le fait que** les électrodes EMS présentent une structure non élastiquement extensible.

9. Vêtement EMS selon l'une quelconque des revendications précédentes, **caractérisé par le fait qu'**au moins chaque section de surface textile (1, 2, 3; 101, 102, 103) portant des électrodes EMS est constituée d'un matériau textile non élastiquement extensible et exempt d'élasthanne.
